# EUROPÄISCHE PATENTSCHRIFT

(11) **EP 1 878 412 B1**
(45) Veröffentlichungstag und Bekanntmachung des Hinweises auf die Patenterteilung: **02.03.2011**
(21) Anmeldenummer: 07012647.9
(22) Anmeldetag: 28.06.2007
(51) Int. Cl.: A61F 9/008, A61B 19/00

(54) **Ophthalmochirurgischer Arbeitsplatz**
Ophthalmosurgical workplace
Poste de travail ophtalmochirurgical

(30) Priorität: 14.07.2006 DE 102006033054
(43) Veröffentlichungstag der Anmeldung: 16.01.2008
(73) Patentinhaber: Carl Zeiss Surgical GmbH, 73447 Oberkochen (DE)
(72) Erfinder: Gaida, Gerhard, Dr., 73430 Aalen (DE); Papke, Gundel, 15236 Lossow (DE); Gieske, Jana, 73430 Aalen (DE); Andrews, Delbert Peter, 73447 Oberkochen (DE); Müller, Christian, Dr., 89129 Langenau (DE); Nahm, Werner, Dr., 74426 Bühlerzell (DE)
(74) Vertreter: Gauss, Nikolai

(56) Entgegenhaltungen:
- WO-A-00/00253
- DE-A1- 1 491 094
- US-A- 4 854 301
- US-A- 5 098 426
- US-A- 5 413 555
- US-A1- 2005 039 567

## Beschreibung

Die Erfindung betrifft einen ophthalmochirurgischen Arbeitsplatz, welcher ein Mikroskop und einen zugehörigen ersten Fußschalter aufweist, wobei das Mikroskop mit dem Fußschalter über eine Konsole verbunden ist.

Bei Operationen in der Augenchirurgie ist es üblich, viele hochentwickelte Geräte einzusetzen. Um dabei eine hohe Sicherheit zu erreichen, ist zur Bedienung der Geräte große Sorgfalt, viel Erfahrung und ein hohes Maß an Vorsicht von den im Operationssaal tätigen Personen erforderlich. Dies betrifft unter anderem Verbindungselemente zwischen den einzelnen Geräten, wie z. B. Anschlusskabel, Schläuche und ähnliches. Es ist von Bedeutung, dass in einem Operationssaal zum Beispiel keine der dort tätigen Personen über derartige Verbindungselemente stolpert, durch eine ungewollte Handbewegung keine Steckverbindung gelöst wird oder zwei nicht zueinander zugehörige Verbindungselemente miteinander gekoppelt werden. Neben einer Unfallgefahr im Operationssaal besteht zusätzlich die Gefahr, dass durch derartige ungewollte Aktionen auch ein Operationserfolg für den Patienten nicht erreicht wird.

Die Komplexität der zu verwendenden Geräte bei einer Augenoperation führt dazu, dass viel Zeit dafür aufgewendet werden muss, um das geforderte hohe Maß an Sorgfalt und Vorsicht bei einer Operation sicher zu stellen. Während eine Katarakt-Operation meist nur etwa 15 Minuten Operationszeit erfordert, muss für die vorbereitenden Maßnahmen und die Sicherheitsmaßnahmen ein ähnlich großer Zeitraum vorgesehen werden. Wenn diese begleitenden Maßnahmen weniger Zeit beanspruchen würden, könnten mehr Operationen in einer Arbeitsschicht durchgeführt und eine höhere Produktivität erreicht werden.

In US 5,098,426 ist eine Operationseinrichtung beschrieben, welche ein Mikroskop, ein an der Basis befestigtes Stativ, einen an der Basis befestigten Stahl sowie einen Fußschalter aufweist, welcher ebenfalls an der Basis montiert ist.

Es ist daher eine Aufgabe der Erfindung, einen Arbeitsplatz vorzuschlagen, mit dem mehr Operationen je Arbeitsschicht bei gleichzeitig mindestens gleich hoher, vorzugsweise noch höherer Arbeitssicherheit erreicht werden kann. Ferner ist es eine Aufgabe der Erfindung, einen ophthalmochirurgischen Arbeitsplatz vorzuschlagen, bei dem ein Operateur weniger zeitaufwändige Nebentätigkeiten und weniger Handgriffe durchführen muss, so dass insgesamt ein entspannteres Arbeiten während einer Operation und einer gesamten Arbeitsschicht möglich ist, wobei gleichzeitig eine große Flexibiltät bei der Anordnung eines zu behandelnden Patienten erreichbar ist.

Die Aufgabe wird durch einen Arbeitsplatz mit den Merkmalen des Anspruchs 1 gelöst. Vorteilhafte Ausführungsformen ergeben sich aus den Merkmalen der abhängigen Ansprüche.

Der ophthalmochirurgische Arbeitsplatz gemäß der Erfindung weist ein Mikroskop und einen zugehörigen ersten Fußschalter auf, wobei das Mikroskop mit dem Fußschalter über eine Konsole verbunden ist, wobei das Mikroskop und der erste Fußschalter grob vorpositioniert sind und eine Änderung der relativen Position zwischen Mikroskop und erstem Fußschalter zueinander nur durch eine Feinpositionierung möglich ist. Damit sind die Freiheitsgrade und der Bewegungsraum des Mikroskopes und des ersten Fußschalters stark eingeschränkt, so dass ein zeitaufwändiges Vorpositionieren nicht mehr erforderlich ist. Die einander zugehörigen Geräte wie Mikroskop und erster Fußschalter sind bereits an einer für den Operateur geeigneten Position, wobei eine Feinpositionierung nur noch für eine individuelle Einstellung je nach Operateur erforderlich ist. Die Vielzahl an Verstellmöglichkeiten für ein Mikroskop und einen Fußschalter, wie sie noch beim Stand der Technik möglich ist, wird gemäß der Erfindung erheblich reduziert. Insbesondere ist ein Vorpositionieren eines z. B. an einer Operationssaaldecke angebrachten Mikroskopes und eines frei im Raum bewegbaren Fußschaltpultes nicht mehr notwendig. Die Feinpositionierung erlaubt nur noch einen kleinen Bewegungsraum für das Mikroskop und den ersten Fußschalter. Dies bedeutet, dass die Geräte nur noch dort vorhanden sind, wo ein Operateur sie benötigt und erwartet, so dass die Unfallgefahr und der potentiell beanspruchte Raum im Operationssaal verringert sind. Somit kann die Aufmerksamkeit des im Operationssaal bei einer Operation tätigen Personals verstärkt auf die zusätzlichen Geräte und die eigentliche Augenoperation gerichtet werden.

Gemäß der Erfindung ist das Mikroskop mit der Konsole über einen von der Konsole abstehenden Haltearm verbunden. Ein abstehender Haltearm ermöglicht es, dass zwischen Mikroskop und Konsole ein genügender Abstand erreicht wird, um direkt unter das Mikroskop einen Patienten auf einer Patientenliege anzuordnen. Die Konsole ist dann seitlich an der Patientenliege vorgesehen, während das Mikroskop mittels des Haltearmes direkt über dem zu operierendem Auge platziert werden kann.

Vorzugsweise ist der Haltearm in der Vertikalen verlagerbar. Damit können unterschiedlich hohe Patientenliegen zum Einsatz kommen und das Mikroskop in der Höhe so eingestellt werden, dass ein Operateur damit entspannt arbeiten kann. Indem der Haltearm in der Vertikalen verlagerbar ist, wird ein Beitrag zur Ergonomie des ophthalmochirurgischen Arbeitsplatzes geschaffen.

Vorzugsweise ist die Konsole, welche das Mikroskop mit dem ersten Fußschalter verbindet, turmartig aufgebaut. Die Konsole übernimmt damit die Funktion, den Höhenunterschied zwischen Fußschaltpult und Mikroskop zu erzielen. Ein Haltearm, an dem das Mikroskop befestigt ist, kann damit so gestaltet werden, dass er z. B. nur als ein im Wesentlichen waagrechtes Teil von der Konsole absteht. Dies ermöglicht einen relativ einfachen und schlanken Aufbau der aus Konsole und Haltearm mit Mikroskop bestehenden Baugruppe.

Es ist vorteilhaft, wenn die Konsole mindestens ein Zusatzgerät aufweist, welches aus der Gruppe ausgewählt ist, welche ein Phakoemulsifikationsgerät, ein Vitrektonomiegerät, ein Laserkoagulationsgerät, einen Endoilluminator und eine Videovorrichtung aufweist, wobei jedes Zusatzgerät mit einem zusätzlichem Fußschalter versehen ist. Es können auch andere Zusatzgeräte, die für die Ophthalmologie benötigt werden, in der Konsole vorgesehen sein. Damit wird erreicht, dass neben der Konsole kein derartiges Zusatzgerät positioniert werden muss. Verbindungskabel, Anschlüsse, Schläuche und ähnliches sind vollständig innerhalb der Konsole untergebracht. Dies verbessert die Arbeitsplatzsicherheit erheblich und verringert eine Unfallgefahr, während gleichzeitig der von den Geräten beanspruchte Platz im Operationssaal auf ein Minimum reduziert werden kann. Der für das Personal verfügbare Platz in einem Operationssaal kann dadurch erheblich vergrößert werden. Durch die Integration von einem oder mehreren Zusatzgeräten in eine Konsole ist es möglich, eine einheitliche Bedienung vorzusehen. Das Einlernen in die Handhabung von Geräten mit gleicher Bedienphilosophie lässt sich dadurch im Vergleich zur Bedienung mehrerer Einzelgeräte mit unterschiedlicher Bedienphilosophie verkürzen. Ferner können dadurch einige Bauteile eingespart werden, die zum Betreiben eines Mikroskopes und mindestens eines Zusatzgerätes gleichermaßen erforderlich sind. Es ist zum Beispiel möglich, dass dadurch nur noch eine zentrale Steuerung, ein Netzteil, ein Fahrgestell, ein elektrischer Anschluss und ein Datenanschluss für den Betrieb des ophthalmochirurgischen Arbeitsplatzes erforderlich sind. Dies verringert die Zahl der Fehlermöglichkeiten und ermöglicht eine kostengünstigere Konstruktion.

Besonders vorteilhaft ist es, wenn der erste Fußschalter und der jeweilige zusätzliche Fußschalter eines Zusatzgerätes auf einem gemeinsamen Fußschaltpult angeordnet sind. Zum einen verringert sich damit der erforderliche Platz in einem Operationssaal im Vergleich zum Platz, den mehrere einzelne Fußschaltpulte benötigen. Zum anderen werden doppelte und mehrfache Verbindungsleitungen eingespart und können in einer einzigen Leitung integriert werden. Auch für den Operateur ist es einfacher, mehrere Fußschalter auf nur einem einzigen Fußschaltpult zu bedienen. Die Verwechslungsgefahr nimmt ab, und zusätzlich kann im Gegensatz zum Stand der Technik eine einheitliche Bedienphilosophie der mehreren Fußschalter vorgesehen werden. Die Kombination aus einer Konsole mit einem daran gekoppelten Mikroskop und mindestens einem der genannten Zusatzgeräte sowie einem gemeinsamen Fußschaltpult erhöht die Integrationsdichte des ophthalmochirurgischen Arbeitsplatzes.

Sind der erste Fußschalter und der jeweilige zusätzliche Fußschalter auf dem Fußschaltpult verschiebbar und zueinander positionierbar angeordnet, kann eine individuelle Anpassung an den jeweiligen Operateur erreicht werden. Gleichwohl handelt es sich hierbei nur um eine Feinpositionierung der jeweiligen Fußschalter. Durch die Anordnung der Fußschalter auf einem gemeinsamen Fußschaltpult sind die Fußschalter bereits grob vorpositioniert. Ein Suchen, Anordnen und Sichern der jeweiligen Fußschalter, wie dies noch bei Lösungen nach dem Stand der Technik erforderlich ist, entfällt bei einer solchen Ausführungsform.

Für eine noch höhere Ergonomie des ophthalmochirurgischen Arbeitsplatzes sind das Fußschaltpult und das Mikroskop zur Konsole bewegbar ausgebildet. Dies kann sowohl in horizontaler als auch in vertikaler Richtung vorgesehen sein. Vorzugsweise sind das Fußschaltpult und das Mikroskop um die Konsole schwenkbar ausgebildet. Damit ist es möglich, das Fußschaltpult und das Mikroskop zum Beispiel um 180° zu schwenken, so dass die Konsole mit einem eventuell enthaltenen Zusatzgerät zum Beispiel nicht mehr rechts, sondern links von einer Patientenliege angeordnet sein kann. Damit wird eine hohe Flexibilität im Hinblick auf eine Positionierung des ophthalmochirurgischen Arbeitsplatzes in einem Operationssaal erreicht. Bei beengten Platzverhältnissen in einem Operationssaal kann somit eine optimale Anordnung des ophthalmochirurgischen Arbeitsplatzes und des für den Operateur am besten geeigneten Sitzplatzes erreicht werden.

Ferner ist es möglich, dass das Mikroskop und die Konsole um das Fußschaltpult schwenkbar ausgebildet sind. Das Fußschaltpult kann somit ortsfest im Operationssaal moniert sein, so dass sich eine noch höhere Flexibilität in der Anordnung des ophthalmochirurgischen Arbeitsplatzes in einem Operationssaal erreichen lässt.

Erfindungsgemäß ist das Fußschaltpult schwenkbar ausgebildet. Damit ist es möglich, dass ein Operateur sowohl längs als auch quer zur Patientenliege (temporal oder superior) eine Operation durchführen kann. Das Fußschaltpult ist dann jeweils in der für die gewählte Operationsposition geeigneten Lage einstellbar. In diesem Zusammenhang ist es geschickt, wenn das Mikroskop und das Fußschaltpult in mindestens zwei vorbestimmte Positionen verlagerbar sind. Damit kann der Operateur zum Beispiel linksseitig oder rechtsseitig vom Patienten die Operation durchführen.

Vorzugsweise ist das Fußschaltpult mit der Konsole mittels eines längs verstellbaren Auslegers verbunden. Dies ermöglicht eine noch bessere Anpassung an die individuellen Verhältnisse eines Operateurs und erhöht die Ergonomie des Arbeitsplatzes.

Vorzugweise weist der Arbeitsplatz einen Monitor und/oder einen Bedienbildschirm auf. Ein im Operationssaal anwesender Assistent kann während einer Operation somit die wichtigsten Daten beobachten und überprüfen. Vor einer Operation können damit bestimmte Profile für einen Operateur oder einen Patienten eingegeben werden. Es kann sich dabei um eine Mikroskopvergrößerung, einen maximalen Druck bei der Zufuhr einer Spülflüssigkeit, eine maximale Energie für einen Hochfrequenzaktuator eines Phakoemulsifikationsgerätes, um eine Lichtintensität bei einem Mikroskop, um Filtereinstellungen oder Einstellungen eines Fußschaltpultes handeln.

Vorzugsweise bilden der Mikroskophaltearm, die Konsole, der Ausleger und das Fußschaltpult in der Seitenansicht im Wesentlichen einen C-förmigen Aufbau. Damit kann oberhalb des Fußschaltpultes mit Ausleger und unterhalb des Haltearmes und Mikroskopes eine Patientenliege mit einem Patienten positioniert werden.

Vorteile und Weiterbildungen der Erfindung werden an Hand der nachfolgenden Figuren erläutert, in denen zeigen:
- Figur 1: eine schematische Darstellung in perspektivischer Vorderansicht eines ophthalmochirurgischen Arbeitsplatzes gemäß der Erfindung;
- Figur 2: eine schematische Darstellung in perspektivischer Rückansicht des ophthalmochirurgischen Arbeitsplatzes gemäß Figur 1;
- Figur 3: eine schematische Darstellung eines ophthalmochirurgischen Arbeitsplatzes in einer Draufsicht, wobei Haltearm und Fußschaltpult in einer ersten Position angeordnet sind;
- Figur 4: eine schematische Darstellung eines ophthalmochirurgischen Arbeitsplatzes in einer Draufsicht, wobei Haltearm und Fußschaltpult in einer zweiten Position angeordnet sind;
- Figur 5: eine schematische Darstellung eines ophthalmochirurgischen Arbeitsplatzes gemäß Figur 4, wobei Mikroskop und Fußschaltpult in einer dritten Position angebracht worden sind; und
- Figur 6: eine schematische Darstellung eines ophthalmochirurgischen Arbeitsplatzes in einer Draufsicht, wobei Mikroskop und Fußschaltpult in einer vierten Position angeordnet sind.

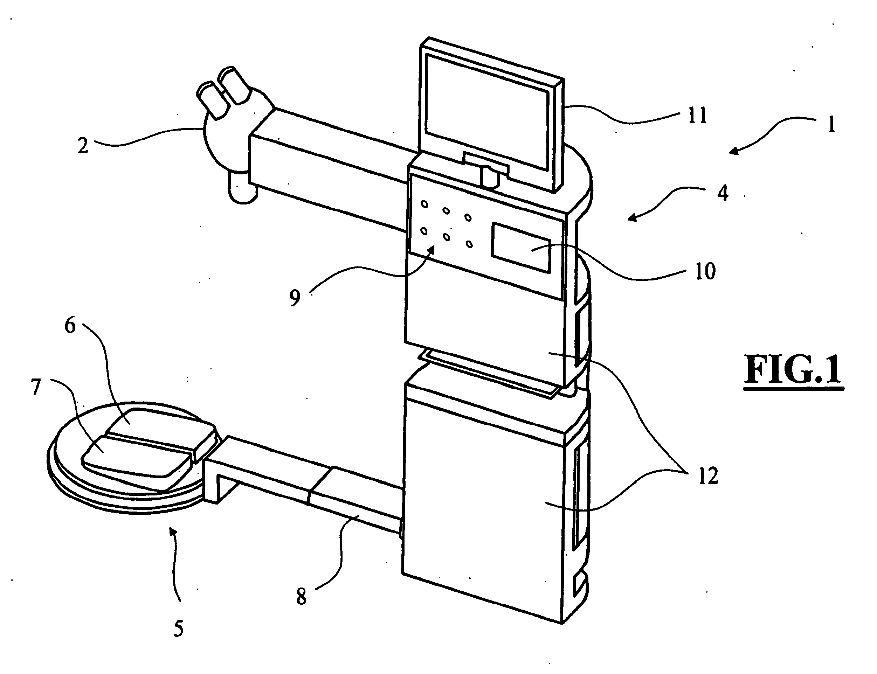

Figur 1 zeigt eine schematische Darstellung eines ophthalmochirurgischen Arbeitplatzes 1 in einer perspektivischen Vorderansicht. Der Arbeitsplatz 1 weist ein Mikroskop 2 auf, welches z.B. ein stereoskopisches Mikroskop mit einer Beleuchtungseinheit ist. Das Mikroskop ist an einem Haltearm 3 befestigt, der mit einer Konsole 4 gekoppelt ist. Die Konsole 4 ist turmartig aufgebaut, wobei in der Konsole eine Vielzahl von Geräten integriert sein kann. Der Arbeitsplatz 1 weist ferner ein Fußschaltpult 5 auf, welches einen ersten Fußschalter 6 und einen zweiten Fußschalter 7 aufweist. Bei einer anderen Ausführungsform als jene, die in Figur 1 dargestellt ist, kann das Fußschaltpult noch zusätzliche Fußschalter aufweisen. Das Fußschaltpult ist mit der Konsole 4 über einen Fußschaltpultausleger 8 gekoppelt. Der Ausleger 8 kann teleskopisch in seiner Längsrichtung verstellbar sein. Damit ist es möglich, das Fußschaltpult 5 an eine für einen Operateur gewünschte Position anzuordnen.

In der Konsole 4 kann z. B. ein Phakoemulsifikationsgerät integriert sein. Bei einem solchen Gerät ist eine Irrigations- und Aspirationseinheit vorgesehen, so dass an einem zu operierenden Auge eine Spülflüssigkeit zugeführt und Augenlinsenfragmente einschließlich Spülflüssigkeit wieder abgesaugt werden können. Anschlüsse für derartige Spül- und Saugleitungen können z. B. im Anschlussfeld 9 vorgesehen sein, welches allgemein für entfernbare sterile Gerätekomponenten vorgesehen ist. Neben Schlauchanschlüssen können dies z. B. Anschlüsse für eine Beleuchtung sein, die für eine Vitrektonomiebehandlung erforderlich ist. Ferner kann das Anschlussfeld 9 allgemein für elektrische und optische Anschlüsse vorbereitet sein. Das Anschlussfeld 9 ist benachbart zu einer auswechselbaren Kassette 10 für eine Pumpvorrichtung in der Konsole 4 platziert. Sowohl das Anschlussfeld 9 als auch die auswechselbare Kassette 10 sind vorzugsweise relativ weit oben in der Konsole 4 platziert. Dies ist für die Bedienung des ophthalmochirurgischen Arbeitplatzes 1 vorteilhaft.

Unterhalb des Anschlussfeldes 9 sind Versorgungsbaugruppen 12 mit Elektronik und Optikvorrichtungen in der Konsole 4 integriert. Oberhalb der Konsole 4 ist ein Monitor 11 angeordnet, der gleichzeitig auch einen Bedienbildschirm aufweisen kann. Der Monitor ist bei der in Figur 1 dargestellten Ausführungsform so angeordnet, dass ein im Operationssaal tätiger Assistent ihn gut einsehen und bedienen kann. Am Monitor 11 kann z.B. das im Mikroskop 2 sichtbare Bild angezeigt werden. Zusätzlich ist es möglich, mit dem Bedienbildschirm (Touch Screen) für eine Augenoperation bestimmte Parameter einzustellen. Die Parameter können durch einen Operateur oder allgemein durch den Operationstyp vorgegeben sein. So ist es z. B. möglich, ein Computerprogramm vorzusehen, mit dem ein bestimmter Datensatz für einen ersten Operateur gespeichert werden kann. Übernimmt ein anderer Operateur die Arbeit an einem solchen Arbeitsplatz, kann ein anderer Datensatz programmiert und / oder aufgerufen werden, der für diesen anderen Operateur geeignet ist. Es kann sich dabei um eine Voreinstellung der Vergrößerung im Mikroskop, um eine bestimmte Lichtintensität und um eine bestimmte Filtereinstellung bei der Beleuchtung des Operationsfeldes handeln. Weitere Parameter können Druckwerte für eine Saug- oder Spülleitung, zur Verfügung gestellte Energie für einen Ultraschallwandler oder Pulsformen zum Antreiben eines Aktuators sein.

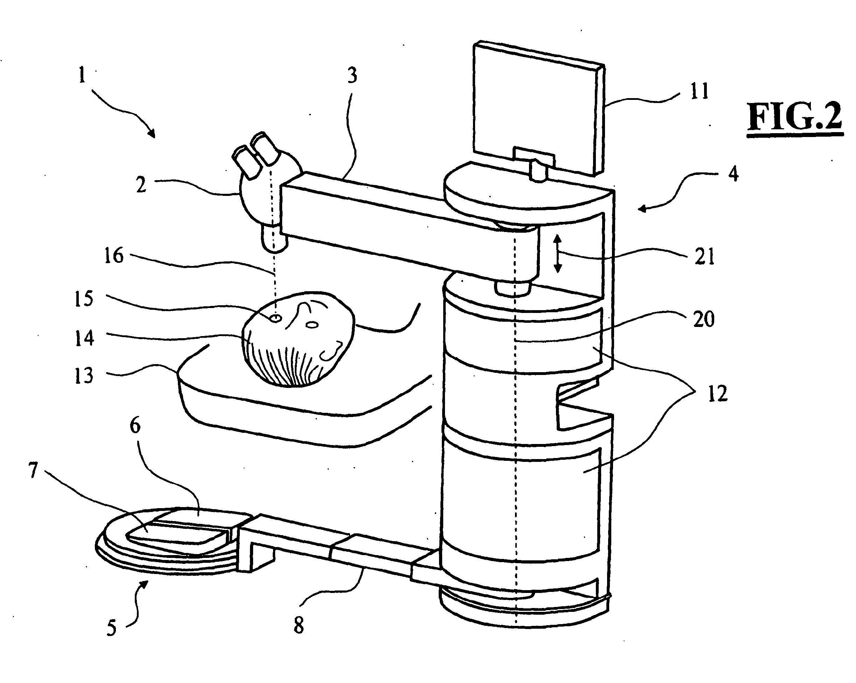

Unterhalb des Mikroskopes 2 und des Haltearmes 3 ist ein Freiraum vorgesehen, der von einer Patientenliege mit einem darauf liegenden Patienten teilweise ausgefüllt werden kann. In Figur 2 ist eine Patientenliege 13 angedeutet, auf der ein Patient 14 derart liegt, dass sich dessen linkes Auge 15 direkt im Strahlengang 16 des Mikroskops 2 befindet. Um den Strahlengang 16 des Mikroskops 2 mit dem Auge 15 des Patienten 14 genau auszurichten, ist bei der Ausführungsform, wie sie in Figur 1 und 2 dargestellt sind, nur ein Verstellen des Haltearms 3 in vertikaler Richtung vorgesehen, siehe Pfeil 21 in Figur 2. Eine Variation der Länge des Haltearmes 3 ist nicht möglich. Ein Ausrichten in horizontaler Richtung erfolgt ausschließlich mittels der Patientenliege 13.

Die Länge des Haltearms 3 und des daran befestigten Mikroskops 2 gibt die Position für einen Operateur vor. Das Fußschaltpult 5 wird dann entsprechend über den Fußschaltpultausleger 8 so positioniert, dass der Operateur entspannt sitzen und durch das Mikroskop 2 hindurch sehen kann. Die Kabel für das Fußschaltpult 5 verlaufen zum Beispiel innerhalb des Auslegers 8. Es ist vorteilhaft, wenn diese Einstellung zwischen Mikroskop 2 und Fußschaltpult 5 bei jedem Operateur speziell ermittelt und anschließend fixiert wird. Es wird darauf hingewiesen, dass die in Fig. 1 und 2 dargestellte teleskopische Verstellmöglichkeit des Auslegers 8 für die praktische Ausführung viel zu groß ist. Der Abstand des Fußschaltpultes 5 zur Konsole 4 ist in der Realität deutlich geringer als der Abstand des Mikroskopes 2 zur Konsole 4. Diese Lage ist durch die Vorpositionierung vorgegeben, für eine Feinpositionierung sind nur noch kleine Verstellmöglichkeiten durchführbar.

Ein Ausrichten des Arbeitsplatzes 1 bezüglich eines zu operierenden Auges 15 ist in horizontaler Richtung ausschließlich mittels der Liege 13 möglich. Durch das Einschränken der Freiheitsgrade und der Bewegungsräume für Mikroskop, Konsole und Fußschaltpult wird Zeit eingespart, um die Geräte zueinander grob vorzupositionieren. Dieses Kriterium ist insbesondere von Bedeutung, da z.B. eine Kataraktoperation üblicherweise nur etwa 15 Minuten dauert, so dass eine Zeitersparnis von wenigen Minuten für ein nicht mehr erforderliches Vorpositionieren der benutzten Geräte zu einer Zeitersparnis führt, welche für zusätzliche Operationen genutzt werden kann. Damit lässt sich durch Einsatz eines erfindungsgemäßen ophthalmochirurgischen Arbeitsplatzes eine höhere Produktivität im Operationssaal erreichen.

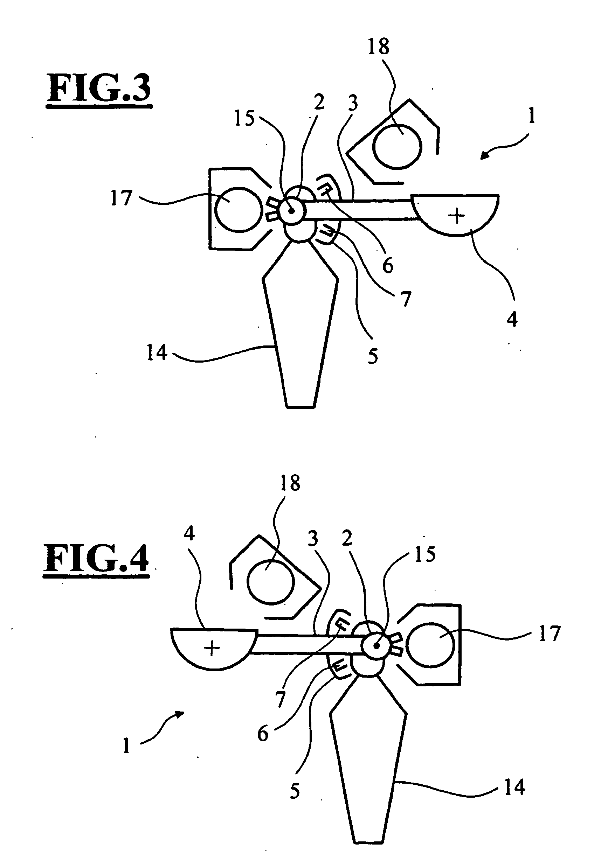

In Figur 3 ist schematisch in einer Draufsicht die Anordnung zwischen ophthalmochirurgischem Arbeitsplatz 1, Patient 14 und Operateur 17 zu erkennen. Der Arbeitsplatz 1 weist die Konsole 4 auf, von welcher ein Haltearm 3 absteht. Am distalen Ende des Haltearms 3 ist ein Mikroskop 2 angeordnet, in dessen Strahlengang das zu operierende Auge 15 des Patienten 14 liegt. Es wird darauf hingewiesen, dass das Auge 15 bei einer Draufsicht gemäß Figur 3 nicht sichtbar ist, hier aber zur besseren Verständlichkeit mit dargestellt ist. Der Operateur 17 sitzt seitlich zur Patientenliege 14 und kann ein Fußschaltpult 5 mit den Fußschaltern 6 und 7 bedienen. Wie aus Figur 2 ersichtlich ist, kann der Haltearm 3 und der Fußschaltpultausleger 8 mit dem Fußschaltpult 5 um eine Achse 20 innerhalb der Konsole 4 geschwenkt werden.

In Figur 4 ist eine Anordnung dargestellt, in der die Konsole 4 an unveränderter Position im Vergleich zu der Anordnung in Figur 3 vorgesehen ist, während der Haltearm 3 und das Fußschaltpult 5 um 180° um die Konsole 4 geschwenkt worden sind. Der Operateur 17 kann bei dieser Anordnung spiegelbildlich zu der in Figur 3 vorgesehenen Anordnung arbeiten. Diese Anordnung kann z. B. gewählt werden, wenn eine Operation nicht am rechten Auge, siehe Figur 3, sondern am linke Auge eines Patienten 14 vorgesehen ist. Ein anderer Grund für die unterschiedlichen Positionen des Mikroskops 2 und Fußschaltpults 5 bezüglich der Konsole 4 können beengte Plätzverhältnisse in einem Operationssaal sein. Zu beachten ist ferner, dass ein Assistent 18 während einer Operation relevante Daten an einem Monitor überwachen oder an der Konsole 4 eine bestimmte Bedienung vornehmen können soll. Bei der in Figur 4 dargestellten Anordnung muss sich der Assistent 18 somit linksseitig von der Patientenliege und vor der Patientenliege bewegen können. Bei der in Figur 3 dargestellten Anordnung muss sich der Assistent 18 rechtsseitig von der Patientenliege und vor der Patientenliege bewegen können. Je nach Arbeitsablauf und räumlichen Beschränkungen in dem Operationssaal kann damit der Arbeitsplatz 1 eingerichtet werden.

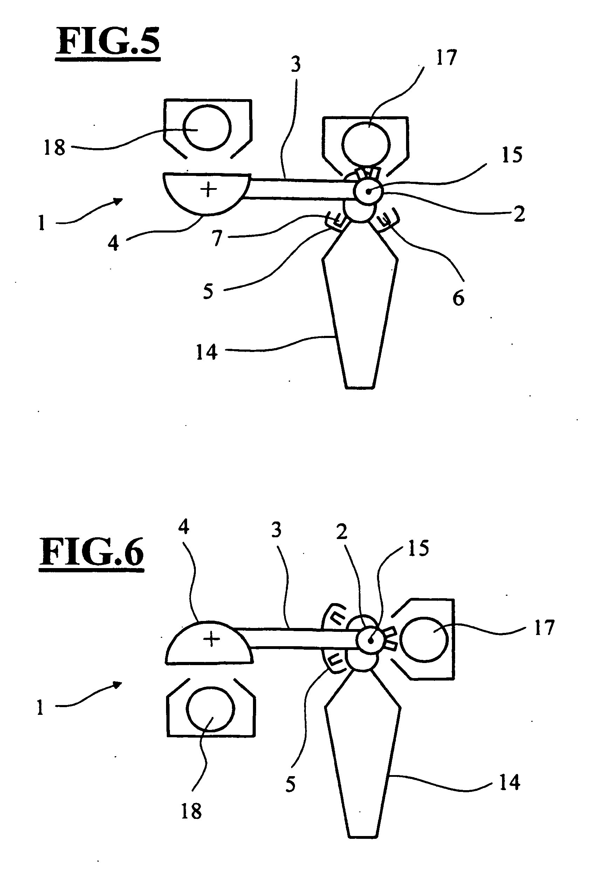

In Figur 5 ist eine weitere Anordnung des Arbeitsplatzes 1 dargestellt. Der Operateur 17 ist hierbei nicht seitlich (temporal) zur Patientenliege 14, sondern in der Verlängerung des Kopfendes der Patientenliege 14 positioniert (superior). Damit auch bei dieser Anordnung das Fußschaltpult 5 bedient werden kann, ist es um eine eigene Achse schwenkbar ausgebildet.

In Figur 6 ist eine weitere Möglichkeit zur Anordnung des Arbeitsplatzes 1 dargestellt. Das Fußschaltpult 5 ist bei dieser Ausführungsform ortsfest am Boden montiert, so dass die Konsole 4 mit dem Haltearm 3 und dem Mikroskop 2 um das Fußschaltpult 5 herum bewegbar sind. Bei dieser Anordnung kann sich der Assistent 18 neben der Patientenliege 14 aufhalten und gleichzeitig den Monitor und/oder Bedienbildschirm 11 sowie die Konsole 4 bedienen. Informationen, die auf dem Monitor 11 zu erkennen sind oder allgemein Parameter für den Betrieb des Arbeitsplatzes darstellen, können durch eine entsprechende Vorrichtung auch für den Operateur in das Mikroskop-Sichtfeld eingebracht werden. Damit kann der Operateur während einer Operation ohne Unterbrechung durch das Mikroskop sehen und muss nicht zur Kontrolle bestimmter Daten zum Monitor aufblicken und dabei die Operation unterbrechen.

## Patentansprüche

1. Ophthalmochirurgischer Arbeitsplatz (1), welcher ein Mikroskop (2) und einen zugehörigen ersten Fußschalter (6) aufweist, wobei das Mikroskop (2) mit dem Fußschalter (6) über eine Konsole (4) verbunden ist, **dadurch gekennzeichnet, dass** das Mikroskop (2) an einem Haltearm (3) befestigt ist, welcher mit der Konsole (4) verbunden ist, dass der erste Fußschalter (6) auf einem Fußschaltpult (5) angeordnet ist und das Fußschaltpult (5) über einem Fußschaltpultausleger (8) mit der Konsole (4) gekoppelt ist, so dass das Mikroskop (2) und der erste Fußschalter (6) grob vorpositioniert sind, und eine Änderung der relativen Position zwischen Mikroskop (2) und erstem Fußschalter (6) zueinander nur durch ein Mittel für eine Feinpositionierung möglich ist, und dass das Fußschaltpult (5) um eine eigene Achse schwenkbar ausgebildet ist.

2. Arbeitsplatz (1) nach Anspruch 1, wobei der Haltearm (3) in der Vertikalen verlagerbar ist.

3. Arbeitsplatz (1) nach einem der Ansprüche 1 bis 2, wobei die Konsole (4) turmartig aufgebaut ist.

4. Arbeitsplatz (1) nach einem der Ansprüche 1 bis 3, wobei die Konsole (4) mindestens ein Zusatzgerät aufweist, welches aus der Gruppe ausgewählt ist, welches ein Phakoemulsifikationsgerät, ein Vitrektonomiegerät, ein Laserkoagulationsgerät, einen Endoilluminator und eine Videovorrichtung aufweist, wobei jedes Zusatzgerät mit einem zusätzlichen Fußschalter (7) versehen ist.

5. Arbeitsplatz nach Anspruch 4, wobei der erste Fußschalter (6) und der jeweilige zusätzliche Fußschalter (7) auf einem gemeinsamen Fußschaltpult (5) angeordnet sind.

6. Arbeitsplatz (1) nach einem der Ansprüche 4 oder 5, wobei der erste Fußschalter (6) und der jeweilige zusätzliche Fußschalter (7) auf dem Fußschaltpult (5) verschiebbar und zueinander positionierbar sind.

7. Arbeitsplatz (1) nach einem der Ansprüche 1 bis 6, wobei das Fußschaltpult (5) und das Mikroskop (2) zur Konsole (4) bewegbar ausgebildet sind.

8. Arbeitsplatz (1) nach einem der Ansprüche 1 bis 7, wobei das Fußschaltpult (5) und das Mikroskop (2) um die Konsole (4) schwenkbar ausgebildet sind.

9. Arbeitsplatz (1) nach einem der Ansprüche 1 bis 7, wobei das Mikroskop (2) und die Konsole (4) um das Fußschaltpult (5) schwenkbar ausgebildet sind.

10. Arbeitsplatz (1) nach einem der Ansprüche 1 bis 9, wobei das Mikroskop (2) und das Fußschaltpult (5) in mindestes zwei vorbestimmte Positionen verlagerbar sind.

11. Arbeitsplatz (1) nach einem der Ansprüche 1 bis 10, wobei das Fußschaltpult (5) mit der Konsole (4) mittels eines längsverstellbaren Auslegers (8) verbunden ist.

12. Arbeitsplatz (1) nach einem der Ansprüche 1 bis 11, wobei der Arbeitsplatz (1) einen Monitor (11) und/oder Bedienbildschirm aufweist.

13. Arbeitsplatz (1) nach einem der Ansprüche 1 bis 12, wobei der Mikroskophaltearm (3), die Konsole (4), der Ausleger (8) und das Fußschaltpult (5) einen in der Seitenansicht im wesentlichen C-förmigen Aufbau bilden.

## Claims

1. Ophthalmic surgical workstation (1), having a microscope (2) and an associated first foot switch (6), the microscope (2) being connected to the foot switch (6) via a console (4), **characterized in that** the microscope (2) is fastened to a holding arm (3) that is connected to the console (4), **in that** the first foot switch (6) is arranged on a foot switch stand (5) and the foot switch stand (5) is coupled to the console (4) via a foot switch stand cross-beam (8) such that the microscope (2) and the first foot switch (6) are roughly pre-positioned, and a change in the relative position between microscope (2) and the first foot switch (6) with respect to one another is possible only by a means for fine positioning, and **in that** the foot switch stand (5) is designed to be able to pivot about a dedicated axis.

2. Workstation (1) according to Claim 1, wherein the holding arm (3) can be displaced in the vertical.

3. Workstation (1) according to one of Claims 1 to 2, wherein the console (4) is designed in the manner of a tower.

4. Workstation (1) according to one of Claims 1 to 3, wherein the console (4) has at least one additional appliance selected from the group comprising a phacoemulsification instrument, a vitrectomy instrument, a laser coagulation instrument, an endoilluminator and a video apparatus, each additional appliance being provided with an additional foot switch (7).

5. Workstation according to Claim 4, wherein the first foot switch (6) and the respective additional foot switch (7) are arranged on a common foot switch stand (5).

6. Workstation (1) according to one of Claims 4 or 5, wherein the first foot switch (6) and the respective additional foot switch (7) can be positioned with respect to one another and such that they can be displaced on the foot switch stand (5).

7. Workstation(1) according to one of Claims 1 to 6, wherein the foot switch stand (5) and the microscope (2) are designed to be moveable with respect to the console (4).

8. Workstation (1) according to one of Claims 1 to 7, wherein the foot switch stand (5) and the microscope (2) are designed such they can pivot about the console (4).

9. Workstation (1) according to one of Claims 1 to 7, wherein the microscope (2) and the console (4) are designed such that they can pivot about the foot switch stand (5).

10. Workstation (1) according to one of Claims 1 to 9, wherein the microscope (2) and the foot switch stand (5) can be displaced into at least two predetermined positions.

11. Workstation (1) according to one of Claims 1 to 10, wherein the foot switch stand (5) is connected to the console (4) by means of a longitudinally adjustable cross-beam (8).

12. Workstation (1) according to one of Claims 1 to 11, wherein the workstation (1) has a monitor (11) and/or user screen.

13. Workstation (1) according to one of Claims 1 to 12, wherein the microscope holding arm (3), the console (4), the cross-beam (8) and the foot switch stand (5) form a construction that is substantially C-shaped in side view.

## Revendications

1. Poste de travail (1) pour chirurgie ophtalmologique, qui présente un microscope (2) auquel est associé un premier commutateur à pédale (6), le microscope (2) étant relié au commutateur à pédale (6) par une console (4),
**caractérisé en ce que**
le microscope (2) est fixé sur un bras de maintien (3) relié à la console (4),
**en ce que** le premier commutateur à pédale (6) est disposé sur un pupitre (5) de commutateurs à pédale,
**en ce que** le pupitre (5) de commutateurs à pédale est raccordé à la console (4) par une flèche (8) de pupitre de commutateurs à pédale, de telle sorte que le microscope (2) et le premier commutateur à pédale (6) soient pré-positionnés grossièrement et qu'une modification de la position relative entre le microscope (2) et le premier commutateur à pédale (6) ne soit possible que par un moyen de positionnement fin, et
**en ce que** le pupitre (5) de commutateurs à pédale est configuré de manière à pouvoir pivoter autour d'un axe qui lui est propre.

2. Poste de travail (1) selon la revendication 1, dans lequel le bras de maintien (3) peut être placé à la verticale.

3. Poste de travail (1) selon l'une des revendications 1 et 2, dans lequel la console (4) a une structure en tour.

4. Poste de travail (1) selon l'une des revendications 1 à 3, dans lequel la console (4) présente au moins un appareil supplémentaire sélectionné dans l'ensemble constitué d'un appareil de phacoémulsification, d'un appareil de vitrectonomie, d'un appareil de coagulation au laser, d'un endo-illuminateur et d'un dispositif vidéo, chaque appareil supplémentaire étant doté d'un commutateur à pédale (7) supplémentaire.

5. Poste de travail selon la revendication 4, dans lequel le premier commutateur à pédale (6) et chaque commutateur à pédale (7) supplémentaire sont disposés sur un pupitre commun (5) de commutateurs à pédale.

6. Poste de travail (1) selon l'une des revendications 4 ou 5, dans lequel le premier commutateur à pédale (6) et chaque commutateur à pédale supplémentaire (7) peuvent être déplacés et positionnés les uns par rapport aux autres sur le pupitre (5) de commutateurs à pédale.

7. Poste de travail (1) selon l'une des revendications 1 à 6, dans lequel le pupitre (5) de commutateurs à pédale et le microscope (2) peuvent être déplacés par rapport à la console (4).

8. Poste de travail (1) selon l'une des revendications 1 à 7, dans lequel le pupitre (5) de commutateurs à pédale et le microscope (2) peuvent pivoter autour de la console (4).

9. Poste de travail (1) selon l'une des revendications 1 à 7, dans lequel le microscope (2) et la console (4) peuvent pivoter autour du pupitre (5) de commutateurs à pédale.

10. Poste de travail (1) selon l'une des revendications 1 à 9, dans lequel le microscope (2) et le pupitre (5) de commutateurs à pédale peuvent être placés dans au moins deux positions prédéterminées.

11. Poste de travail (1) selon l'une des revendications 1 à 10, dans lequel le pupitre (5) de commutateurs à pédale est relié à la console (4) au moyen d'une flèche (8) ajustable en longueur.

12. Poste de travail (1) selon l'une des revendications 1 à 11, dans lequel le poste de travail (1) présente un moniteur (11) et/ou un écran de conduite.

13. Poste de travail (1) selon l'une des revendications 1 à 12, dans lequel le bras de maintien (3) du microscope, la console (4), la flèche (8) et le pupitre (5) de commutateurs à pédale forment une structure essentiellement en forme de C en vue latérale.
